# EUROPÄISCHE PATENTANMELDUNG

(11) **EP 1 671 600 A1**
(43) Veröffentlichungstag der Anmeldung: **21.06.2006**
(21) Anmeldenummer: 05027166.7
(22) Anmeldetag: 13.12.2005
(51) Int. Cl.: A61C 1/05, A61C 1/08, A61B 17/16

(54) **Einsatzteil für einen medizinischen Handstückkopf**

(30) Priorität: 14.12.2004 AT 20932004
(71) Anmelder: W & H Dentalwerk Bürmoos GmbH, 5111 Bürmoos (AT)
(72) Erfinder: Teufelberger, Gunter, 5111 Bürmoos (AT)

(57) **Zusammenfassung**

Die vorliegende Erfindung betrifft ein Einsatzteil (10, 30) für einen medizinischen Handstückkopf (1) zur Aufnahme eines Lagers (23) und von zumindest Teilen der Werkzeugaufnahme (25, 26) sowie zur Übernahme zumindest eines Spraymediums. Das erfindungsgemäße Einsatzteil (10, 30) und bevorzugt auch die Sprayplatte (6 A) als Teil des Einsatzteils (10, 30) sind aus einem elastisch komprimierbaren Material, bevorzugt Kunststoff oder Gummi, besonders bevorzugt Silikon, hergestellt ist. Damit kann das erfindungsgemäße Einsatzteil (10, 30) mehrere Funktionen erfüllen, für die bisher weitere Bauteile benötigt wurden, wie z.B. die Übernahme und Vermischung der Spraymedien (Sprayflüssigkeit, Sprayluft) zur Erzeugung des Behandlungssprays, die Aufnahme, Fixierung und Lagerung des Wälzlagers (23), das die Werkzeugaufnahme (25, 26) des Handstückkopfs (1) lagert, die Abdichtung der Kanäle, Mischkammern (14, 17, 17 A, 19) etc. der Spraymedien sowie das Vorspannen des Wälzlagers (23). Zusätzlich vereinfacht sich der Einbau des Einsatzteils (10, 30) in den Handstückkopf (1).

## Beschreibung

Die vorliegende Erfindung betrifft ein Einsatzteil für einen medizinischen Handstückkopf zur Abgabe eines Behandlungssprays.

Ein derartiges aus Metall gefertigtes Einsatzteil ist beispielhaft aus der EP 109 507 A1 bekannt. Es ist im Inneren des Handstückkopfs aufgenommen und ragt mit seinem werkzeugseitigen Ende aus dem Kopf heraus. Rund um die Seitenflächen des Einsatzteils verlaufen eine erste Ringnut, in die eine Sprayflüssigkeit führende Medienleitung mündet, und eine zweite Ringnut, in die eine Sprayluft führende Medienleitung mündet. Von diesen beiden Ringnuten verlaufen Bohrungen durch das Einsatzteil bis zur werkzeugseitigen Oberfläche des Einsatzteils, von wo die Sprayflüssigkeit und die Sprayluft auf das Werkzeug und die Behandlungsstelle abgegeben werden.

Das Einsatzteil ist mittels Presssitz mit dem äußeren Laufring des Kugellagers verbunden. Andere der Anmelderin bekannte Einsatzteile werden im Handstückkopf verklebt oder verschraubt. Das Kugellager ist in einer Schulter des Einsatzteils aufgenommen und über (in der EP 109 507 A1 nicht dargestellte) Federscheiben vorgespannt. Mehrere O-Ringe dichten die Ringnute und Medienleitungen gegen den Innenraum des Handstückkopfs ab.

Der vorliegenden Erfindung liegt die Aufgabe zugrunde, einen Einsatzteil für einen medizinischen Handstückkopf zu verbessern, wobei insbesondere eine Reduktion der zusätzlich benötigten Bauteile (O-Ringe, Federscheibe) und ein vereinfachter Einbau des Einsatzteils in den Handstückkopf angestrebt werden.

Diese Aufgabe wird gemäß der vorliegenden Erfindung durch ein Einsatzteil mit den Merkmalen des Anspruchs 1 gelöst. Besonders vorteilhafte Ausführungsformen sind in den Unteransprüchen angeführt.

Durch die Herstellung aus einem elastisch komprimierbaren Material, bevorzugt Kunststoff oder Gummi, besonders bevorzugt Silikon, kann das Einsatzteil zusätzliche Funktionen übernehmen, so dass es insbesondere dient:
- zur Übernahme und Vermischung der Spraymedien (Sprayflüssigkeit, Sprayluft) zur Erzeugung des Behandlungssprays.
- zur Aufnahme, Fixierung und Lagerung des Wälzlagers, das die Werkzeugaufnahme des Handstückkopfs lagert.
- zur Abdichtung der Kanäle, Mischkammern etc. der Spraymedien.
- zum Vorspannen des Wälzlagers (in Abhängigkeit der Elastizität des gewählten Materials).

In vorteilhafter Weise werden somit keine Dichtelemente oder Federelemente mehr benötigt. Auch ist der Einbau in den Handstückkopf erleichtert, da, im Gegensatz zu den bisherigen Einsatzteilen, ein Verkleben oder Verschrauben im Handstückkopf nicht mehr notwendig ist.

Das Einsatzteil hat bevorzugt eine zylindrische oder zylindrisch-konische Außenform und folgt damit der Innengeometrie der Außenhülse des Handstückkopfs bzw. ist deren Form und Abmessungen angepasst. Aufgrund der elastischen Materialeigenschaften und insbesondere aufgrund der radialen Elastizität erfährt das Einsatzteil, wenn das Wälzlager in das Einsatzteil eingesetzt wird, eine radiale Verbreiterung (Vergrößerung des Durchmessers), wodurch es mit seiner Mantelfläche gegen die Innenseite der Außenhülse des Handstückkopfs gedrückt und im Handstückkopf geklemmt wird. Dadurch wird zusätzlich eine hohe Dichtwirkung erzielt, so dass keine Gefahr besteht, dass Sprayluft oder Sprayflüssigkeit entweicht.

In einem besonders bevorzugten Ausführungsbeispiel ist auch die Sprayplatte zur Abgabe des Sprays Teil das Einsatzteils und aus einem elastisch komprimierbaren Material, bevorzugt aus Kunststoff oder Gummi, besonders bevorzugt aus Silikon, hergestellt. Hierdurch wird insbesondere der Montageaufwand für den Handstückkopf weiter verringert. Zusätzlich lösen sich Kalkablagerungen selbsttätig von der Sprayplatte, wenn diese durch Druckausübung verformt wird.

Die Erfindung wird nachfolgend anhand bevorzugter Ausführungsbeispiele und Bezug nehmend auf die beigefügten Zeichnungen erläutert:
Figur 1 zeigt einen Handstückkopf mit einem ersten Ausführungsbeispiel des erfindungsgemäßen Einsatzteils.
Figur 2 zeigt einen Handstückkopf mit einem zweiten Ausführungsbeispiel des erfindungsgemäßen Einsatzteils.
Figur 3 zeigt eine perspektivische Darstellung des erfindungsgemäßen Einsatzteils.
Figur 4 zeigt eine Schnittdarstellung des erfindungsgemäßen Einsatzteils.

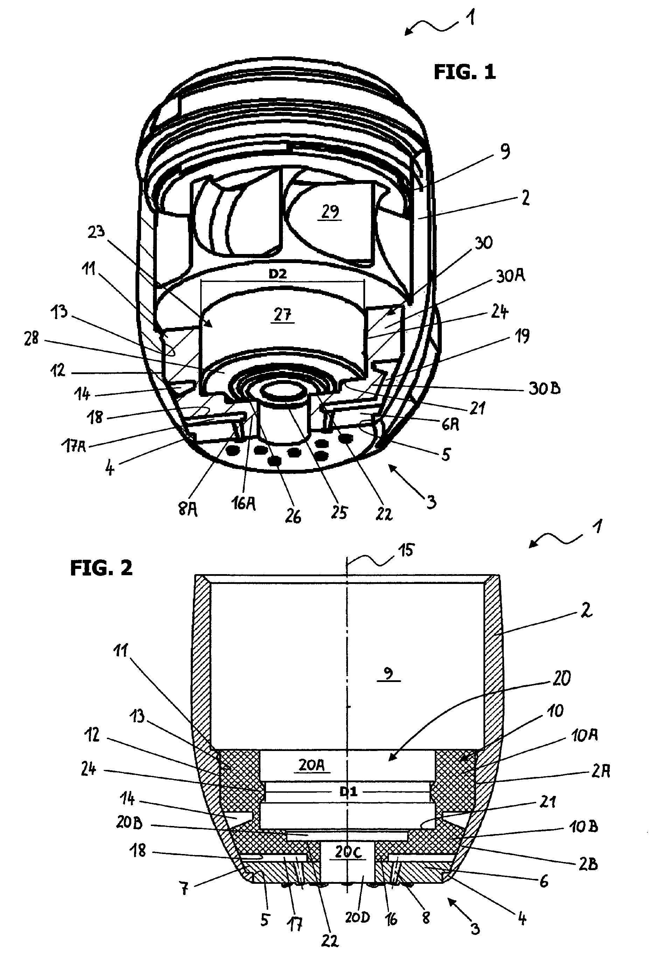

Der in Figur 2 dargestellte Handstückkopf 1 ist Teil eines gewinkelten medizinischen Handstücks (Winkelstücks), das aus unterschiedlichen medizinischen Anwendungen bekannt ist. Im Handstück ist ein Laufrad 29 (siehe Figur 1) angeordnet, das über Druckluft in Rotation versetzt wird. Mit dem Laufrad 29 ist eine Werkzeugaufnahme verbunden, in die ein Werkzeug, zum Beispiel ein Dentalbohrer, eingesetzt ist und mit dem Laufrad 29 in Rotation versetzt wird. Der Handstückkopf 1 besteht aus einer Außenhülse 2, die an ihrem werkzeugseitigen Ende 3 eine Schulter 4 aufweist. In Öffnung 5 des Handstückkopfs 1 ist eine flache, kreisförmige Sprayplatte 6 eingesetzt, die aus Kunststoff oder Metall besteht. Sprayplatte 6 weist an ihrer Rückseite einen Flansch 7 auf, der an der Schulter 4 anliegt, wodurch die Sprayplatte 6 in der Öffnung 5 gelagert und positioniert wird. Die Sprayplatte 6 ist von mehreren Bohrungen 8 durchsetzt, über die das Spray (Sprayflüssigkeits-Sprayluft-Gemisch) an die Behandlungsstelle und ein in die Werkzeugaufnahme des Handstückkopfs 1 eingesetztes Werkzeug abgegeben wird. Bevorzugt weisen die Bohrungen 8 eine leichte Neigung in Richtung der Mittelachse 15 auf, so dass die abgegebene Sprayflüssigkeit auf das Werkzeug gerichtet wird. Sind mehrere Bohrungen 8 vorhanden, so können diese unterschiedliche Neigungen aufweisen, um sicher zu stellen, dass bei Verwendung von Werkzeugen unterschiedlicher Längen Sprayflüssigkeit genau auf die Spitze des Werkzeugs und die diese umgebende Behandlungsstelle abgegeben wird.

Im Innenraum 9 des Handstückkopfs 1 ist, anschließend an die Sprayplatte 6, ein Einsatzteil 10 angeordnet. Das Einsatzteil 10 besteht aus einem elastisch komprimierbaren Material, bevorzugt Kunststoff oder Gummi, besonders bevorzugt Silikon. Das Einsatzteil 10 ist in seiner äußeren Form der Innengeometrie der Außenhülse 2 des Handstückkopfs 1 angepasst: Es weist einen zylindrisch geformten Abschnitt 10 A auf, entsprechend einem zylindrisch geformten Abschnitt 2 A der Innenwand 13 der Außenhülse 2 und einen sich konisch verjüngenden Abschnitt 10 B, entsprechend einem sich konisch verjüngend geformten Abschnitt 2 B der Innenwand 13 der Außenhülse 2.

Einsatzteil 10 weist an seiner Mantelfläche 12 einen Ringwulst 11 auf, dessen Durchmesser so bemessen ist, dass, wenn Einsatzteil 10 in den Handstückkopf 1 eingesetzt ist, der Ringwulst 11 an der Innenwand 13 der Außenhülse 2 dichtend anliegt, wodurch ein Eindringen von Sprayflüssigkeit und -luft in den oberhalb des Einsatzteils 10 gelegenen Bereich des Innenraums 9 unterbunden wird. Eine Ringnut 14 umläuft das Einsatzteil 10 vollständig. Diese Ringnut 14 ist mit einer ersten Medienleitung verbunden, bevorzugt einer Sprayluftleitung (nicht dargestellt), die in bekannter Weise durch das gesamte Handstück verläuft und mit einer Druckluftquelle verbunden ist.

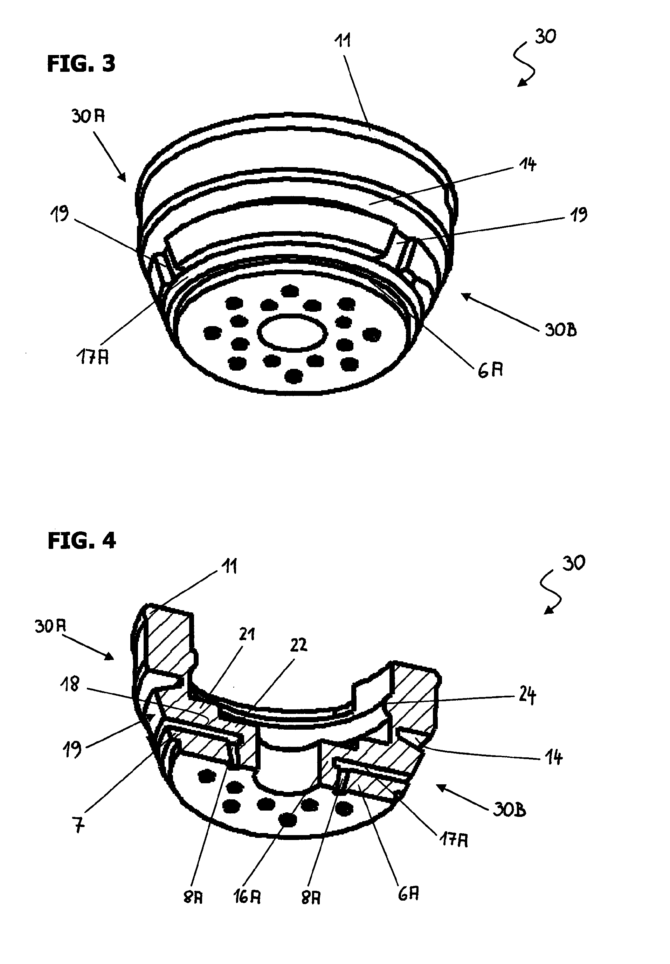

Das der Öffnung 5 zugewandte Ende des Einsatzteils 10 wird durch einen Ringwulst 16 gebildet, der unmittelbar der Sprayplatte 6 aufsitzt. Durch den Ringwulst 16 wird ein Ringspalt 17 zwischen der Sprayplatte 6 und der Schulter 18 des Einsatzteils 10 geschaffen. Dieser Spalt 17 ist mit einer zweiten Medienleitung verbunden, bevorzugt einer Sprayflüssigkeitsleitung (nicht dargestellt), die ebenfalls in bekannter Weise durch das gesamte Handstück verläuft und mit einer Sprayflüssigkeitsquelle, bevorzugt einem Anschluss an eine Wasserleitung, verbunden ist. Ringspalt 17 und Ringnut 14 sind über einen oder mehrere Verbindungskanäle 19 (siehe Figuren 1, 3 und 4) miteinander verbunden. Bei Betrieb des Handstücks fließt die Sprayluft von Ringnut 14 über Verbindungskanal 19 in den Spalt 17 und vermischt sich dort mit der Sprayflüssigkeit. Ringspalt 17 dient somit auch als Mischkammer für das abzugebende Spray aus Sprayflüssigkeit und Sprayluft. Das Spray wird anschließend über die Bohrungen 8, die mit einem ihrer Enden in den Ringspalt 17 münden, nach außen abgegeben.

Der Innenraum 20 des Einsatzteils 10 umfasst drei Abschnitte 20 A, 20 B und 20 C, die durch zwei Schultern 21 und 22 voneinander getrennt sind. Abschnitt 20 A dient der Aufnahme eines Lagers 23, bevorzugt eines Wälzlagers / Kugellagers (siehe Figur 1), und bildet mit Schulter 21 einen Lagersitz. Lager 23 lagert in bekannter Weise die rotierende Spannzange 25 zur lösbaren Aufnahme und Fixierung des Bearbeitungswerkzeugs und die ebenfalls rotierende Spindel 26, auf welcher das Laufrad 29 angeordnet ist. Kugellager 23 wird durch einen Ringwulst 24, der in den Innenraum 20 des Einsatzteils 10 ragt, fixiert. Der Durchmesser D1 des Innenraumabschnitts 20 A im Bereich des Ringwulsts 24 ist etwas geringer als der Außendurchmesser D2 des Kugellagers 23. Dadurch wird eine sichere Befestigung des Kugellagers 23 im Einsatzteil 10 garantiert. Gleichzeitig bewirkt das Einsetzen des Lagers 23 in den Lagersitz 20 A, 21 aufgrund der elastischen Materialeigenschaften und insbesondere aufgrund der radialen Elastizität eine radiale Verbreiterung (Vergrößerung des Durchmessers) des Einsatzteils 10, wodurch dieses mit seiner Mantelfläche 12 fest an die Innenseite 13 der Außenhülse 2 gedrückt und im Handstückkopf 1 geklemmt wird. Dadurch wird zusätzlich eine hohe Dichtwirkung erzielt, so dass keine Gefahr besteht, dass Sprayluft oder Sprayflüssigkeit aus der Ringnut 14 oder dem Spalt 17 entweicht.

Wie insbesondere aus Figur 1 ersichtlich ist, lagert der äußere Laufring 27 als nicht bewegter Teil des Wälzlagers 23 gemeinsam mit einer Abdeckscheibe 28 direkt auf der Schulter 21 des Einsatzteils 10. Der rotierende innere Laufring des Kugellagers 23 (nicht zu erkennen) sowie die ebenfalls rotierende Werkzeugaufnahme (Spannzange 25, Spindel 26) sind durch den Abschnitt 20B des Einsatzteils 10 von der Schulter 22 beabstandet, so dass kein Kontakt zwischen bewegten und nicht bewegten Bauteilen vorliegt.

Die Abschnitte 20A, 20B und 20C des Innenraums 20 bilden auch eine Durchführung zur Aufnahme des Werkzeugschafts und von zumindest Teilen der Werkzeugaufnahme (Spannzange 25, Spindel 26). Die Durchführung setzt sich in einer Bohrung 20D, die die Sprayplatte durchsetzt, fort.

Das in den Figuren 1, 3 und 4 dargestellte Einsatzteil 30 gleicht in seinem Aufbau und in seiner Funktion dem Einsatzteil 10, so dass von einer nochmaligen Beschreibung der gleichen Bauteile Abstand genommen wird. Im Unterschied zu Einsatzteil 10 ist bei Einsatzteil 30 jedoch auch die Sprayplatte 6 A als Teil des Einsatzteils 30 ausgebildet und besteht ebenfalls aus einem elastisch komprimierbaren Material, bevorzugt aus Kunststoff oder Gummi, besonders bevorzugt aus Silikon. Sprayplatte 6 A ist über einen Steg 16 A mit den Abschnitten 30 B und 30 A des Einsatzteils 30 verbunden. Eine Ringnut 17 A zwischen der Sprayplatte 6 A und der Schulter 18 nimmt ein Medium, bevorzugt die Sprayflüssigkeit, aus einer Medienleitung auf und dient gleichzeitig wiederum als Mischkammer. Mehrere Bohrungen 8 A zur Abgabe des Sprays durchsetzten die Sprayplatte 6 A und münden in der Ringnut 17 A. Bevorzugt weisen die Bohrungen 8 A eine leichte Neigung in Richtung der Mittelachse 15 auf, so dass die abgegebene Sprayflüssigkeit auf das Werkzeug gerichtet wird. Sind mehrere Bohrungen 8 A vorhanden, so können diese unterschiedliche Neigungen aufweisen, um sicher zu stellen, dass bei Verwendung von Werkzeugen unterschiedlicher Längen Sprayflüssigkeit genau auf die Spitze des Werkzeugs und die diese umgebende Behandlungsstelle abgegeben wird. Die Positionierung und Lagerung der Sprayplatte 6 A in der Öffnung 5 des Handstückkopfs 1 erfolgt in gleicher Weise wie bei der Sprayplatte 6 mittels eines Flansches 7, der an der Schulter 4 der Außenhülse 2 anliegt.

In einem weiteren Ausführungsbeispiel sind im Einsatzteil 10, 30 ein oder mehrere Stütz- und Versteifungselement, bevorzugt Stützringe, Halbschalen oder Buchsen aus Metall oder Hartkunststoff, angeordnet, die die Stabilität des Einsatzteils 10, 30 erhöhen. Diese Elemente können sowohl nach der Herstellung des Einsatzteils 10, 30 (bevorzugt im Spritzgussverfahren) in Ausnehmungen (Rücksprünge, Nuten) des Einsatzteils 10, 30 eingefügt werden oder bevorzugt vollständig im Einsatzteil 10, 30 aufgenommen sein, wenn sie schon vor der Herstellung in die Spritzgussform eingefügt wurden. Durch Einsetzen eines Federelements, bevorzugt einer Federscheibe, in eine zusätzliche Nut im Einsatzteil 10, 30, unterhalb des Lagersitzes 20 A, 21 des Lagers 23, wird die Vorspannung für das Lager 23 erhöht.

Die Erfindung ist nicht auf das dargestellte Anwendungsgebiet und die beschriebenen Ausführungsbeispiele beschränkt, sondern umfaßt alle Ausführungsmöglichkeiten, die das prinzipielle, sinngemäße Funktionsprinzip der Erfindung nicht verändern.

## Patentansprüche

1. In einen medizinischen Handstückkopf (1) einsetzbares Einsatzteil (10, 30) zur Aufnahme eines Lagers (23) und von zumindest einem Teil der Werkzeugaufnahme (25, 26) des medizinischen Handstückkopfs (1) sowie zur Übernahme zumindest eines Spraymediums mit einem Lagersitz (20A, 21) für ein Lager (23) und mit zumindest einer Ringnut (14) in der Mantelfläche (12) des Einsatzteils (10, 30), die mit einer ersten Medienleitung zur Übergabe eines Spraymediums verbindbar ist,
**dadurch gekennzeichnet, dass**
das Einsatzteil (10, 30) aus einem elastisch komprimierbaren Material, bevorzugt Kunststoff oder Gummi, besonders bevorzugt Silikon, hergestellt ist.

2. Einsatzteil (10, 30) nach Anspruch 1, **dadurch gekennzeichnet, dass**
das Einsatzteil (10, 30) eine zweite Ringnut (17A) umfasst, die mit einer zweiten Medienleitung zur Übergabe eines Mediums verbindbar ist, wobei das in die erste Ringnut (14) eingeleitete Medium Sprayluft und das in die zweite Ringnut (17A) eingeleitete Medium eine Sprayflüssigkeit ist.

3. Einsatzteil (10, 30) nach Anspruch 1 oder 2, **gekennzeichnet durch**
zumindest einen Verbindungskanal (19) zur Verbindung der ersten Ringnut (14) mit der zweiten Ringnut (17A) oder der ersten Ringnut (14) mit einer Sprayplatte (6, 6A).

4. Einsatzteil (10, 30) nach einem der vorhergehenden Ansprüche, **dadurch**
**gekennzeichnet, dass**
das Einsatzteil (10, 30) der Innengeometrie der Außenhülse (2) des Handstückkopfs (1) folgt und bevorzugt aus einem zylindrischen Abschnitt (10 A, 30 A) und einem sich konisch verjüngenden Abschnitt (10 B, 30 B) gebildet ist.

5. Einsatzteil (10, 30) nach einem der vorhergehenden Ansprüche, **dadurch**
**gekennzeichnet, dass**
das Einsatzteil (10, 30) einen Innenraum (20) mit mehreren Abschnitten (20 A, 20 B, 20 C) umfasst, die durch Schultern (21, 22) voneinander getrennt sind.

6. Einsatzteil (10, 30) nach Anspruch 5, **dadurch gekennzeichnet, dass**
der Lagersitz (20A, 21) im Innenraum (20) angeordnet ist und einen Ringwulst (24) umfasst, wodurch der Durchmesser (D1) des Innenraums (20) im Bereich des Ringwulsts (24) geringer ist als der Durchmesser (D2) des im Lagersitz (20A, 21) aufgenommenen Lagers (23).

7. Einsatzteil (10, 30) nach einem der vorhergehenden Ansprüche, **gekennzeichnet**
**durch**
einen Ringwulst (11) an der Mantelfläche (12) des Einsatzteils (10, 30) zur Abdichtung des an das Einsatzteil (10, 30) anschließenden Innenraums (9) des Handstückkopfs (1).

8. Einsatzteil (10, 30) nach einem der vorhergehenden Ansprüche, **dadurch**
**gekennzeichnet, dass**
im Einsatzteil (10, 30) ein oder mehrere Feder-, Stütz- oder Versteifungselement, bevorzugt Federscheiben, Stützringe, Halbschalen oder Buchsen aus Metall oder Hartkunststoff, angeordnet sind.

9. Einsatzteil (10, 30) nach einem der vorhergehenden Ansprüche, **dadurch**
**gekennzeichnet, dass**
das Einsatzteil (10, 30) insbesondere eine radiale Elastizität aufweist, die bewirkt, dass, ein in den Lagersitz (20A, 21) eingesetztes Lager (23) das Einsatzteil (10, 30) mit seiner Mantelfläche (12) dichtend gegen die Innenseite (13) der Außenhülse (2) des Handstückkopfs (1) drückt und im Handstückkopf (1) festklemmt.

10. Einsatzteil (10, 30) nach einem der vorhergehenden Ansprüche, **dadurch**
**gekennzeichnet, dass**
die Sprayplatte (6 A) Teil des Einsatzteils (10, 30) ist und aus einem elastisch komprimierbaren Material, bevorzugt aus Kunststoff oder Gummi, besonders bevorzugt aus Silikon, hergestellt ist.

11. Einsatzteil (10, 30) nach Anspruch 10, **gekennzeichnet durch**
einen Flansch (7) an der Sprayplatte (6 A) zum Positionieren der Sprayplatte (6 A) an einer Schulter (4) der Außenhülse (2) des Handstückkopfs (1).

12. Einsatzteil (10, 30) nach Anspruch 10 oder 11, **dadurch gekennzeichnet, dass**
die Sprayplatte (6 A) zumindest eine Bohrung (8 A) zur Sprayabgabe umfasst, wobei die Bohrung (8 A) eine Neigung in Richtung der Mittelachse 15 aufweist, so dass von der Sprayplatte (6 A) abgebbare Sprayflüssigkeit auf ein in der Werkzeugaufnahme (25, 26) des Handstückkopfs (1) aufnehmbares Werkzeug gerichtet ist.
